# EUROPÄISCHE PATENTSCHRIFT

(11) **EP 2 589 346 B1**
(45) Veröffentlichungstag und Bekanntmachung des Hinweises auf die Patenterteilung: **23.04.2014**
(21) Anmeldenummer: 12190758.8
(22) Anmeldetag: 31.10.2012
(51) Int. Cl.: A61B 17/122

(54) **Vorgespannter Aneurysmenclip**
Pre-tensioned aneurysm clip
Clip d'anévrisme précontraint

(30) Priorität: 07.11.2011 DE 102011055094
(43) Veröffentlichungstag der Anmeldung: 08.05.2013
(73) Patentinhaber: AESCULAP AG, 78532 Tuttlingen (DE)
(72) Erfinder: Motz, Corvin, 88630 Pfullendorf (DE); Zieris, Gerold, 78570 Mühlheim (DE); Amann, Joachim, 78357 Mühlingen-Zoznegg (DE)
(74) Vertreter: Winter, Brandl, Fürniss, Hübner, Röss, Kaiser, Polte - Partnerschaft

(56) Entgegenhaltungen:
- DE-U1-202010 008 512
- GB-A- 2 161 206
- US-A- 4 765 335
- US-A1- 2010 004 678

## Beschreibung

Die vorliegende Erfindung betrifft einen chirurgischen Clip und insbesondere einen Aneurysmenclip gemäß dem Oberbegriff des Anspruchs 1.

Chirurgische Clips (oder auch Gewebeclip genannt) sind medizinische Implantate, die zumeist temporär zum Verschließen beispielsweise von Gewebeperforationen oder zur langzeitlichen Therapie von Aneurysmen als Gefäßklammern eingesetzt werden. Hierfür eignen sich ein Vielzahl von Clipformen beginnend mit maulartigen Clips, bei denen die Klemm- oder Clipbranchen ähnlich zu einem Ober- und Unterkiefer als zwei einander gegenüberliegende, längs gebogene Balken oder Zahnreihen ausgeformt sind, die an ihren jeweiligen beiden Längsenden über (zwei) Scharniere miteinander gekoppelt sind, bis hin zu reihenartigen (geraden) Clips, die vergleichbar zu einer herkömmlichen Zange zwei sich gegenüberliegende im Wesentliche gerade Klemmschienen haben, die ggf. mit Zähnen oder einer Riffelung besetzt sind und die nur an jeweils einem Längsende miteinander scharnierartig gekoppelt sind.

Allen bekannten Clipformen jedoch gemein ist zumindest eine Federanordnung, die eine Klemmkraft auf die Clipbranchen anlegt. Diese zumindest eine Federanordnung kann als ein separates Bauteil ausgeführt sein, das in den Clip eingesetzt ist, um die Clipbranchen gegeneinander vorzuspannen oder sie ist in den Clip (einstückig) integriert. Im letzteren Fall bildet die Federanordnung im Wesentlichen das Scharnier bzw. die Scharniere, mit welchem(n) die beiden Clipbranchen schwenkbar gekoppelt sind.

Aus dem Stand der Technik ist ein chirurgischer Clip der Gattung mit geraden Clipbranchen und endseitiger Federanordnung bekannt, wie er beispielsweise in der DE 20 2010 008 512 U1 veröffentlicht ist.

Die Klemm- oder Schließkraft wird hier durch eine Schenkelfeder erzeugt, die entweder als Rund- oder Rechteckfeder ausgebildet ist. Im Konkreten besteht ein solcher Clip aus zwei Clipbranchen, die sich in ihren jeweiligen Mittenabschnitten lose (d.h. ohne mechanische Verbindung wie z.B. ein Scharnierstift) überkreuzen, um zwei in Schließposition parallel aneinanderliegende Klemmabschnitte und zwei in dieser Position voneinander beabstandete Betätigungsschenkel bzw. Betätigungsabschnitte zu bilden. Die freien Enden der Betätigungsabschnitte sind über die vorstehend genannte Schenkelfeder miteinander verbunden, die entweder einstückig (aus einem einzigen Teil) mit den Clipbranchen oder als separates Bauteil ausgeformt und anschließend mit den Clipbranchen zu einem Teil verbunden (verlötet, verschweißt, zusammengesteckt, etc.) ist. Die Schenkelfeder kann hierbei entweder eine halbe, eine 1.5-fache oder sogar eine 2-fache Wicklung besitzen.

Zur Betätigung wird der chirurgische Clip an seinen Betätigungsabschnitten zusammengedrückt, wodurch sich die Klemmabschnitte aufgrund der sich überkreuzenden Ausrichtung der Clipbranchen voneinander beabstanden. Gleichzeitig wird die Schenkelfeder stärker vorgespannt. Werden die Betätigungsabschnitte freigegeben, bewirkt die Schenkelfeder ein Auseinanderdrücken der Betätigungsabschnitte, bis die Klemmabschnitte gegeneinander gepresst sind.

Obgleich die vorstehende Konstruktion eine ausreichend hohe Anpresskraft zwischen den beiden Klemmabschnitten gewährleistet und die Schenkelfeder aufgrund eines nur geringen elastischen Deformationsgrads wenig belastet wird und daher sehr haltbar ist, ergeben sich doch einige Nachteile.

So erfordert die Verwendung der bekannten Schenkelfeder einen vergleichsweise aufwändigen und schwierigen Herstellungsvorgang für das Wickeln der Feder, das ohne Rissbildung des Federmaterials erfolgen muss. Auch besteht hierbei die Möglichkeit eines Überwickelns der Schenkelfeder, wodurch sich die Anpresskraft zwischen den Klemmabschnitten verringert. Insgesamt bedarf die Herstellung der Schenkelfeder daher ein hohes Maß an Präzision, um Instrumente herzustellen, deren Eigenschaften (Anpresskraft, Haltbarkeit, Betriebssicherheit etc,) innerhalb eines engen Toleranzbereichs liegen. Die Ausschussquote ist daher entsprechend hoch. Grundsätzlich ergeben sich neben den vorstehend genannten spezifischen Nachteilen auch generelle Probleme bei der Verwendung einer Schenkelfeder.

Die Schenkelfeder muss so gestaltet und dimensioniert sein, dass eine vorbestimmte Vorspannkraft auf die Klemmabschnitte ausgeübt wird, die ausschlaggebend ist für die maximal erreichbare Schließkraft. Bei vorgegebenen äußeren Abmessungen des Clip wird jedoch die notwendige Schließkraft häufig nicht erreicht oder es tritt eine plastische Verformung des Federbereichs auf. Des Weiteren können im Fall einer asymmetrischen Anordnung einer separaten Schenkelfeder Reibungskräfte zwischen den Klemmabschnitten und der Feder auftreten, welche die maximal erreichbare Schließkraft weiter mindern. Außerdem kann es sein, dass die Klemmabschnitte nicht parallel öffnen/schließen.

Aus einem weiteren Stand der Technik gemäß der DE 20 2010 008 714 U1 ist ein chirurgischer Clip bekannt mit zwei Clipbranchen, die sich parallel zueinander erstrecken ohne sich zu kreuzen und die in ihren jeweiligen Mittenbereichen über eine stegförmige Biegefeder miteinander verbunden sind. Die Clipbranchen sind demzufolge über die Biegefeder in deren Längsrichtung hinaus verlängert und bilden so zwei Klemmabschnitte auf der einen Seite der Biegefeder und zwei Betätigungsschenkel / -abschnitte auf der anderen Seite der Biegefeder, etwa nach dem Prinzip einer allgemein bekannten Wäscheklammer. Werden demnach die beiden Betätigungsabschnitte auf der einen Seite der stegförmigen Biegefeder gegeneinander gedrückt, bewegen sich die beiden Klemmabschnitte auf der anderen Seite der Biegefeder voneinander weg und umgekehrt.

Dieser chirurgische Clip ist aus einem einzigen Teil, beispielsweise als Stanz- oder Laserteil gefertigt und ist damit hinsichtlich seiner Herstellung relativ preisgünstig. Durch den Stanz- oder Laserschneidvorgang ist im Gegensatz zum vorstehend beschriebenen Clip die Fertigung im Wesentlichen maschinell durchführbar und damit äußerst präzise. Die Biege- und Federkraftverläufe sind auch bei großen Stückzahlen ohne Aufwand innerhalb einer vergleichsweise engen Toleranz reproduzierbar. Damit kann auf einfache Weise ein hoher Qualitätsstandart erreicht werden. Aber auch diese Lösung hat Nachteile.

Obgleich die Biegefeder eine offene, in Richtung hin zu den Betätigungsabschnitten ausbauchende Ringform aufweist, ist der Deformationsweg/-grad der Biegefeder im normalen Gebrauch vergleichsweise groß, sodass die Feder schnell ermüden kann.

Außerdem ist die Biegespannung gegenüber der Schenkelfeder viel höher, wodurch auch häufiger Federbrüche möglich sind. Dies resultiert letztlich in einer gegenüber der Schenkelfeder geringeren Betriebssicherheit und Betriebsdauer.

Ausgehend von der DE 20 2010 008 512 U1 ist es die Aufgabe der vorliegenden Erfindung, einen vergleichsweise preiswerten chirurgischen Clip insbesondere Aneurysmenclip mit verbesserten Eigenschaften bereitzustellen. Ein Ziel ist es hierbei, dass der Clip eine hohe Betriebssicherheit aufweist und möglichst vielseitig einsetzbar ist. Ein weiteres/anderes Ziel ist es, dass der Clip einfach herstellbar/montierbar ist. Zudem sollte der Clip vorzugsweise einen (integrierten) Schutzmechanismus vor mechanischer Überlastung beispielsweise in Folge von unsachgemäßer Handhabung haben.

Diese Aufgabe wird durch einen chirurgischen Clip insbesondere Aneurysmenclip mit den Merkmalen des Anspruchs 1 gelöst. Vorteilhafte Ausgestaltungen der Erfindung sind Gegenstand der Unteransprüche.

Ein erster unabhängiger Aspekt der Erfindung sieht demnach vor, den chirurgischen Clip und insbesondere den Aneurysmenclip aus wenigstens zwei Einzelbauteilen zu fertigen, umfassend eine erste, vorzugsweise eine gerade Klemmschiene aufweisende Branche, an der (vorzugsweise an deren einem proximalen Endbereich) ein Gabel- oder Ösenabschnitt oder ein Anlenkarm (in Form einer dezentralen Einarm-Schwinge) an-/ausgeformt oder anschließbar ist und eine hierzu separate zweite, vorzugsweise eine gerade Klemmschiene aufweisende Branche, an der (vorzugsweise an deren einem proximalen Endbereich) eine Spiralfeder an-/ausgeformt oder anschließbar ist, die mit ihren Seitenflächen in den Gabel-/Ösenabschnitt relativ bewegbar eingesetzt ist oder längs des Anlenkarms geführt ist und die mit dem Gabel-/Ösenabschnitt oder Anlenkarm vorzugsweise (im Wesentlichen) in deren Zentrum/Auge (zumindest in Aufweitrichtung der Spiralfeder drehfest) gekoppelt oder koppelbar ist.

Durch die Verwendung einer (vorzugsweise flachen, bandförmigen) Spiralfeder als das Mittel zur Vorspannung der beiden Branchen sowie zur Festlegung/Einstellung der maximalen Schließkraft ergibt sich ein vergleichsweise großer Federweg auch bei kleiner äußerer Abmessung der Spiralfeder, wodurch sich der Clip weit öffnen lässt, ohne die Feder plastisch zu überbiegen oder zu brechen. Des Weiteren stellt der Gabel-/Ösenabschnitt oder Anlenkarm eine seitliche (vorzugsweise beidseitige) Führung für die Spiralfeder dar, die somit bei einer elastischen Deformation (Aufweitung der Spiralfeder in Radialrichtung) nicht seitlich ausbauchen oder verkanten kann. Dies bewirkt, dass sich die Branchen des chirurgischen Clip im Wesentlichen parallel öffnen und wieder schließen. Schließlich ergibt sich aus der getrennten/separaten Fertigung der beiden Branchen die Möglichkeit, die Spiralfeder und/oder den Gabel-/Ösenabschnitt bzw. Anlenkarm jeweils einstückig sowie vorzugsweise in einem Arbeitsgang, beispielsweise durch Laserschneiden oder Stanzen mit der jeweiligen Branche d.h. dem Klemmabschnitt/Klemmschiene auszuformen, wodurch sich insgesamt das Herstellungsverfahren vereinfacht und gleichzeitig die Herstellungspräzision erhöht. Auch kann sich hierdurch ein bestimmter Federdrahtquerschnitt, beispielsweise eine Rechteckform, ergeben, der einem seitlichen Ausbauchen der Feder unter Biegespannung zusätzlich entgegenwirkt.

Vorzugsweise hat die Spiralfeder gemäß der Erfindung 1.5 bis 2 Windungen, um einen ausreichenden Öffnungswinkel für die beiden Branchen/Klemmschienen sicher zu gewährleisten. Da die Spiralfeder, wie vorstehend angedeutet wurde, durch einen maschinellen (Material abtragenden) Fertigungsprozess wie Schneiden, Stanzen, etc. herstellbar ist, können kleine Fertigungstoleranzen bei geringer Ausschussrate realisiert werden, was den Clip insgesamt preisgünstiger macht, die Stückzahl erhöht und die Betriebssicherheit steigert.

Ein anderer ggf. unabhängiger oder zusätzlicher Aspekt der Erfindung sieht vor, die beiden Branchen vorzugsweise endseitig über eine/die (gemäß dem vorstehenden Aspekt) Spiralfeder elastisch/flexibel zu koppeln, die an ihrem einen, äußeren Ende mit der zweiten Branche (fest) verbunden/verbindbar ist und an ihrem anderen, inneren Ende (Zentrum/Auge) mit der ersten Branche (zumindest in Aufweitrichtung der Spiralfeder drehfest) verbunden/verbindbar ist und an der ein Rastvorsprung (oder - rücksprung) ausgeformt ist, der zur Definition einer maximal zulässigen (aufweitenden) Deformation der Spiralfeder mit einem Anschlag an jener ersten Branche in Anlage kommt, die am inneren Ende der Spiralfeder (drehfest) angeschlossen ist.

Wie vorstehend bereits ausgeführt wurde, erlaubt die Spiralfeder einen weiten elastischen Deformationsweg insbesondere in Aufweitrichtung ohne Bruchgefahr oder plastische Deformation. Um dennoch die Funktionsfähigkeit der Spiralfeder auch bei unsachgemäßer Handhabung zu garantieren, ist diese mit dem Rastvor-/-rücksprung versehen, der vorzugsweise an der radialen Oberseite einer äußeren Windung der Spiralfeder angeordnet ist. Bei einer Relativverschwenkung der durch die Spiralfeder gekoppelten Branchen/Klemmschienen bewegt/dreht sich die erste, im Zentrum/Auge der Spiralfeder gekoppelte Branche im Wesentlichen auf einer Kreisbahn längs der äußeren Windung der Spiralfeder weg von der anderen zweiten Branche hin zu dem Rastvor-/-rücksprung, wobei letztlich der Gabel-/Ösenabschnitt bzw. Anlenkarm mit diesem in Anlage/Anschlag kommt. Damit ist der maximale Relativ-Verdrehwinkel in Öffnungsrichtung des Clip zwischen den beiden Branchen durch den Rastvor-/- rücksprung auf einfache Weise begrenzt und ein Überbiegen der Spiralfeder sicher ausgeschlossen.

Ein anderer ggf. unabhängiger oder zusätzlicher Aspekt der Erfindung sieht vor, die beiden Branchen vorzugsweise endseitig über eine/die (gemäß zumindest einem der vorstehenden Aspekte) Spiralfeder elastisch/flexibel zu koppeln, die an ihrem einen, äußeren Ende mit der zweiten Branche (fest) verbunden/verbindbar ist und an ihrem anderen, inneren Ende (Zentrum/Auge) mit der ersten Branche (zumindest in Aufweitrichtung der Spiralfeder drehfest) verbunden/verbindbar ist. Die (drehfeste) Verbindung zwischen der ersten Branche und der Spiralfeder in deren Zentrum/Auge erfolgt mittels eines vorzugsweise separaten, oder an der ersten Branche fixierten Bolzens, der in übereinander sich anordnender Durchgangsbohrungen im Zentrum der Spiralfeder sowie in der ersten Branche bzw. dem Gabel-/Ösenabschnitt oder Anlenkarm (reibschlüssig) eingesteckt ist und der vorzugsweise einen in Drehrichtung (zumindest in Aufweitrichtung der Spiralfeder) wirkenden Formschluss mit der Spiralfeder und der ersten Branche bzw. dem Gabel-/Ösenabschnitt oder Anlenkarm eingeht. Zusätzlich kann der Bolzen mit der ersten Branche bzw. dem Gabel-/Ösenabschnitt oder Anlenkarm und/oder der Spiralfeder verklebt, verschweißt oder verstemmt sein.

Dies hat den Vorteil, dass beide separate (getrennt voneinander hergestellte) Branchen einschließlich der Spiralfeder bzw. dem Gabel-/Ösenabschnitt oder Anlenkarm einfach miteinander verbolzt werden können, um den Clip fertig zu stellen. Hierbei ist die separate (getrennte) Fertigung der beiden Branchen einfacher und preisgünstiger als die gemeinsame Fertigung aus einem Stück, insbesondere dann, wenn als Vorspannmittel eine Spiralfeder vorgesehen ist. Diese kann dann zusammen mit der zweiten Branche beispielsweise aus einem Stück aus einem Rohling geschnitten oder gestanzt werden.

Alternativ zu der vorstehenden Variante eines im Wesentlichen drehfest eingebauten zentralen Bolzens besteht gemäß einem weiteren ggf. unabhängigen oder zusätzlichen Aspekt grundsätzlich auch die Möglichkeit, einen zweiten Bolzen oder Mitnahmestift vorzugsweise seitlich an der Spiralfeder in radialem Abstand zum zentralen Bolzen zu fixieren oder auszuformen, der unter Ausbildung eines Hebelarms mit dem Gabel-/Ösenabschnitt oder Anlenkarm bei einer Aufweitbewegung für die Spiralfeder in Anlage kommt und so diese aufbiegt.

Gemäß einem anderen ggf. unabhängigen oder zusätzlichen Aspekt der Erfindung bildet der Gabel- oder Ösenabschnitt einen Aufnahmespalt für die Spiralfeder aus, dessen Spaltweite so dimensioniert ist, dass die Spiralfeder seitlich in Gleitkontakt mit den Gabel- oder Ösenabschnitt kommt oder ein geringes Spaltmaß ausbildet, derart, dass der Gabel- oder Ösenabschnitt eine Führungs- und/oder Abstützfunktion für die Spiralfeder ausübt. Somit wird die Aufweitung der Spiralfeder bei Auseinanderbewegen der zwei Klemmschienen geführt und ein Verkanten vermieden. Die trägt dazu bei, dass die theoretisch mögliche Vorspannkraft der Spiralfeder im Betrieb auch tatsächlich erreicht wird.

Gemäß einer ggf. unabhängig zu beanspruchenden vorteilhaften Ausbildung der Erfindung kann es vorgesehen sein, dass die (quer) verlaufenden Durchgangsöffnungen in der Spiralfeder und dem Gabel-/Ösenabschnitt oder dem Anlenkarm ein unrundes bzw. vielnutiges Querschnittsprofil für eine Formschlussverbindung zumindest in Aufweitrichtung der Feder aufweisen. Ferner kann es vorgesehen sein, dass in unvorgespannter Konstruktionslage der bereits ineinander gesteckten jedoch noch nicht gekoppelten Branchen die unrunde Durchgangsöffnung in der Spiralfeder um ein vorbestimmtes Winkelmaß verdreht zu den unrunden Durchgangsöffnungen im Gabel-/Ösenabschnitt oder Anlenkarm ist, derart, dass ein gemeinsames Durchdringen aller Durchgangsöffnungen mittels eines entsprechend unrunden/vielnutigen Bolzens nur durch vorspannendes Verdrehen der Spiralfeder zur Erreichung einer Deckungslage ermöglicht ist. Somit kann entweder eine bestimmte oder eine entsprechend dem Vielnutprofil gestuft bzw. variabel wählbare Vorspannung erreicht/eingestellt werden.

Auch wäre es vorteilhaft, wenn die Spiralfeder einstückig mit der einen zweiten Clipbranche vorzugsweise durch ein Laser-Schneidverfahren oder ein Stanzverfahren ausgebildet ist, da eine solche maschinelle Herstellung einfach und preisgünstig sowie sehr präzise ist. Hierbei kann sich vorzugsweise ein Federdrahtquerschnitt einstellen, der eine Rechtecksform hat. Diese verhindert besonders ein Verdrillen des Federdraht und damit ein Verkanten der Klemmschienen.

Die Erfindung wird nachstehend anhand bevorzugter Ausführungsbeispiele unter Bezugnahme auf die begleitenden Figuren näher erläutert.
Fig. 1 zeigt die Perspektivenansicht eines chirurgischen Clips, insbesondere Aneurysmenclips gemäß einem ersten bevorzugten Ausführungsbeispiel der Erfindung,
Fig. 2 zeigt die vergrößerte Perspektivenansicht des erfindungsgemäßen Vorspannmittels in Form einer Spiralfeder zur Verbindung und Vorspannung zweier Clipbranchen des chirurgischen Clips gemäß der Erfindung,
Fig. 3 zeigt eine Unteransicht des erfindungsgemäßen chirurgischen Clips,
Fig. 4 zeigt eine Längsansicht des erfindungsgemäßen chirurgischen Clips in Konstruktionslage und
Fig. 5 zeigt die Perspektivenansicht eines chirurgischen Clips, insbesondere Aneurysmenclips gemäß einem zweiten bevorzugten Ausführungsbeispiel der Erfindung,

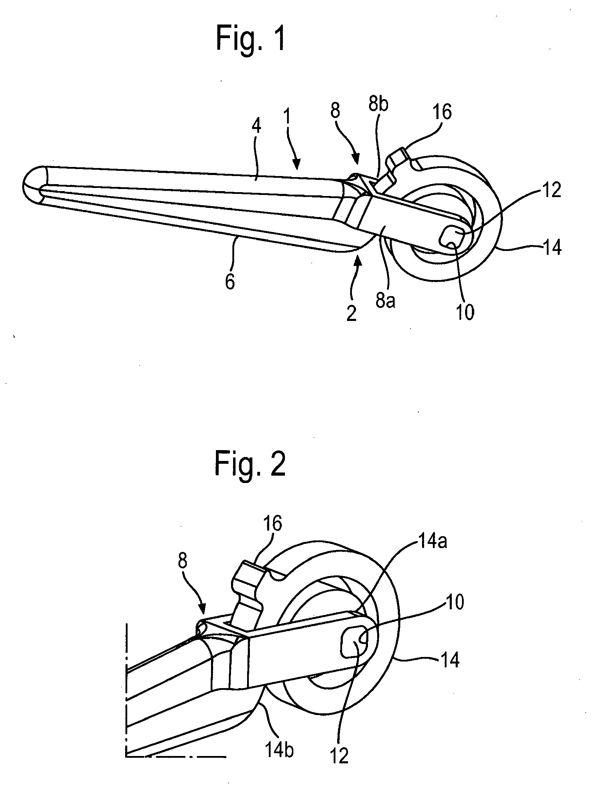

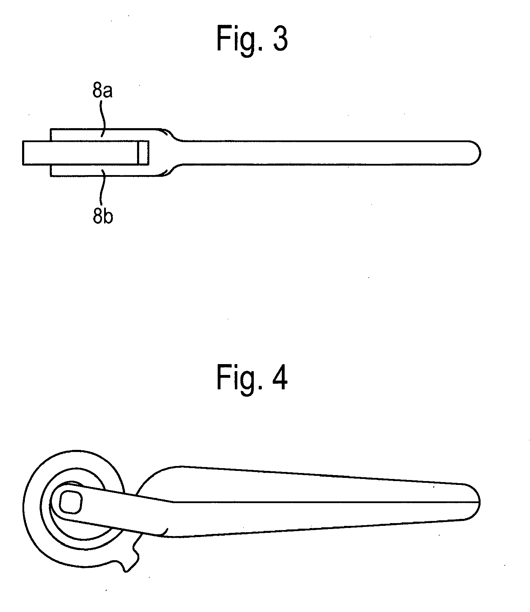

Gemäß der Fig. 1 hat ein chirurgischer Clip gemäß einem ersten bevorzugten Ausführungsbeispiel der Erfindung zwei Clipbranchen 1, 2 jeweils bestehend aus einer vorzugsweise geraden Klemmschiene 4, 6, die an ihren sich zugewandten Längsseiten jeweils eine kooperierende (in gemeinsame Anlage bringbare) Klemmfläche ausbilden. Die eine (gemäß der Fig. 1 obere/erste) Clipbranche 1 hat an einem proximalen Ende der Klemmschiene 4 eine vorliegend gegabelten Lagerachse oder Schwinge 8 vorzugsweise einstückig mit der Klemmschiene 4 bestehend aus zwei im Wesentlichen parallelen, plattenförmigen Lagerarmen 8a, 8b (siehe insbesondere Fig. 3), deren jeweilige Flachseiten einander zugewandt sind, wodurch sich zwischen den Lagerarmen 8a, 8b ein Aufnahmespalt ergibt. Jede der beiden Lagerarme 8a, 8b ist mit einer Durchgangsöffnung 10 endseitig (d.h. im jeweils proximalen Endabschnitt jedes Lagerarms 8a, 8b) versehen, die fluchtend zueinander ausgerichtet sind. Alternativ zu der in der Fig. 1 dargestellten konstruktiven Ausführung der Lagerachse 8 kann diese auch aus einem zentralen Lagersockel oder Schiene bestehen, in welcher ein durchgehender Längsschlitz stellvertretend zu dem in den Fig. 1 bis 4 gezeigten Aufnahmespalt eingearbeitet ist, wodurch der Lagersockel oder Schiene eine Art Öse mit zwei parallel verlaufenden Ösenflanken ausformt. Weiter alternativ kann die Lagerachse aber auch als ein einziger Lagerarm ausgebildet sein, der zu einer gedachten Längsmittellinie der Klemmschiene parallel versetzt (asymmetrisch) platziert ist, wodurch sich ein fluchtend zur gedachten Längsmittellinie verlaufender zentraler Aufnahmebereich parallel zum einzigen Lagerarm ergibt. Diese Form eines einzigen Lagerarms entspricht somit quasi der allgemein bekannten Einarmschwinge beispielsweise einer bekannten Motorradaufhängung. Sowohl im Fall der Ösenform als auch im Fall der Einarmschwingen-Konstruktion sind die Arme mit quer verlaufenden Durchgangsöffnungen versehen, wie es insbesondere auch in der Fig. 2 dargestellt ist.

Im vorliegenden ersten Beispiel hat jede Durchgangsöffnung 10 in den Lagerarmen 8a, 8b einen rechteckigen (quadratischen) Querschnitt, wodurch über einen darin eingesetzten / einsetzbaren Bolzen 12 ein Drehmoment auf die obere Clipbranche 1 übertragbar ist. Alternativ hierzu ist aber auch ein anderes Querschnittsprofil (außer einer symmetrischen Kreisform) denkbar, um in zumindest eine Drehrichtung der Durchgangsöffnung 10 einen Formschluss mit einem darin eingesetzten Bolzen 12 einzugehen. Weiter alternativ kann auch eine Nut- und Federverbindung oder eine Einwegkupplung wie z.B. Ratschenkupplung vorgesehen sein.

An dieser Stelle sei noch darauf hingewiesen, dass die Lagerachse (oder Schwinge) 8 bezüglich der zugehörigen geraden Klemmschiene 4 geringfügig gekröpft bzw. leicht abgewinkelt ist und zwar in eine Richtung, dass die Lagerachse 8 einen stumpfen Winkel zur Klemmfläche der oberen (ersten) Clipbranche 1 eingeht. Ferner sei erwähnt, dass die einstückige Ausbildung von Klemmschiene 4 und Lagerachse 8 nicht unbedingt erforderlich ist, sondern dass beide Abschnitte der oberen Clipbranche 8 auch getrennt (ggf. aus unterschiedlichen Materialien) gefertigt und dann zusammengefügt sein können, beispielsweise durch Schweißen oder Löten. Schließlich ist es grundsätzlich auch möglich, dass die Lagerachse 8 in einem Mittenabschnitt der Klemmschiene 4 angeordnet ist und sich im Wesentlichen rechtwinklig zu dieser erstreckt.

Die andere (gemäß der Fig. 1 untere/zweite) Clipbranche 2 hat gemäß vorstehender Beschreibung ebenfalls die eine, vorliegend gerade Klemmschiene 6, an deren proximalem Ende sich eine Spiralfeder 14 vorzugsweise einstückig anschließt. Im Konkreten hat die Spiralfeder mehrere (vorzugsweise 1.5 bis 3) Federwindungen, wodurch sich ein Zentrum oder Federauge 14a sowie ein äußeres, freies Federende 14b ausbilden. An diesem freien Federende 14b ist die Spiralfeder 14 mit der Klemmschiene 6 unter Ausbildung eines Knicks oder konkaven Abwinklung bezüglich deren Klemmfläche verbunden. Durch diesen Abwinklung wird erreicht, dass das innere Zentrum bzw. Auge 14a der Spiralfeder 14 im Wesentlichen auf einer gedachten Mittellinie der zugehörigen Klemmschiene 6 zu liegen kommt. Im Zentrum 14a der Spiralfeder 14 ist ebenfalls eine (nicht weiter dargestellte) quer verlaufende Durchgangsöffnung ausgeformt mit einem Durchmesser und Querschnitt, der im Wesentlichen den Durchgangsöffnungen 10 der Lagerachse/Schwinge 8 entspricht.

Die einstückige Ausbildung der unteren Klemmschiene 6 und der Spiralfeder 14 hat den Vorteil einer einfachen, vorzugsweise maschinellen (Material abtragenden/schneidenden) Herstellung beispielsweise durch ein Stanz- oder Laserschneidverfahren. Es besteht aber grundsätzlich auch die Möglichkeit, die Klemmschiene sowie die Feder getrennt voneinander (ggf. aus verschiedenen Materialien) herzustellen und wie vorstehend bereits ausgeführt zusammen zu fügen. Auch kann die Spiralfeder in einem Mittenabschnitt der Klemmschiene 6 angeordnet sowie im Wesentlichen rechtwinklig zur Klemmschiene 6 ausgerichtet sein.

Wie insbesondere aus der Fig. 2 zu entnehmen ist, hat die Spiralfeder 14 in einem vorbestimmten Umfangsabstand zu dem Knick bzw. zum freien äußeren Ende 14b einen radialen Vorsprung 16 an deren äußerer Federwindung, der einen Anschlag für die Lagerachse 8 der einen, gemäß Fig. 1 oberen (ersten) Clipbranche 1 zur Begrenzung einer federelastischen Aufbiegung der Spiralfeder 14 bildet, wie dies nachfolgend noch beschrieben wird. Es sei jedoch darauf hingewiesen, dass der Vorsprung 16 auch seitlich (axial) an der Spiralfeder, vorzugsweise an der äußeren Windung angebracht bzw. ausgebildet sein kann.

Die Fig. 1 und 4 zeigen den chirurgischen Clip gemäß dem bevorzugten ersten Ausführungsbeispiel der Erfindung in fertig montiertem Zustand sowie in Konstruktionslage, in welcher die beiden Klemmschienen 4, 6 an deren Klemmflächen mit einer bestimmten Vorspannung aneinander liegen.

Wie hieraus zu ersehen ist, umgreift die gabelförmige Lagerachse oder Schwinge 8 die Spiralfeder 14 beidseits der Federwindungen derart, dass deren proximalen Durchgangsöffnungen 10 mit der nicht gezeigten Durchgangsöffnung im inneren Zentrum 14a der Spiralfeder 14 fluchten. Die Durchgangsöffnung der Spiralfeder 14 ist jedoch in Konstruktionslage (ungespannt) bezüglich der Durchgangsöffnungen 10 der Lagerachse 8 geringfügig nacheilend verdreht, sodass die Spiralfeder 14 für ein Erreichen einer im Wesentlich exakten Deckung / Überlappung aller Durchgangsöffnungen etwas gedreht/aufweitend angespannt werden muss. Erst in diesem Zustand ist es möglich, einen Bolzen 12 mit entsprechenden Querschnittsprofil in die Durchgangsöffnungen 10 der Lagerachse 8 und in die in der Perspektive nicht darstellbare Durchgangsbohrung der Spiralfeder 14 vorzugsweise reibschlüssig zu treiben, wodurch sich nach Freigeben der Spiralfeder 14 über den in Drehrichtung wirkenden Formschluss des Bolzens 12 eine Vorspannung auf die beiden Klemmschienen 4, 6 ergibt.

Je nach Winkelversatz der Durchgangsöffnungen in der Spiralfeder 14 und der Lagerachse 8 gemäß vorstehender Beschreibung können somit unterschiedliche Vorspannkräfte auf die Klemmschienen 4, 6 voreingestellt werden. Dabei liegen die beiden parallelen platten- oder laschenförmigen Arme 8a, 8b nahezu spielfrei (gleitend) an den Seiten der Spiralfeder 14 an, wie dies insbesondere in der Fig. 3 gezeigt ist und stützen diese somit quasi als Führung seitlich ab.

Die Funktionsweise des chirurgischen Clip gemäß dem ersten bevorzugten Ausführungsbeispiel der Erfindung lässt sich wie Folgt beschreiben:
Die gabelförmige Lagerachse 8 befindet sich in montiertem Zustand zwischen der gemäß Fig. 1 unteren Klemmschiene 6 bzw. den Knick 14b zwischen unterer Klemmschiene 6 und Spiralfeder 14 und dem radialen Vorsprung 16 unter Ausbildung eines Abstands zum Vorsprung 16 in Umfangsrichtung der Spiralfeder 14. Werden nunmehr die beiden Klemmschienen 4, 6 auseinander gedrückt, wandert die gabelförmige Lagerachse 8 im Wesentlich entlang der äußeren Windung der Spiralfeder 14 in deren Umfangsrichtung, bis sie am radialen Vorsprung 16 anschlägt und somit die Öffnungsbewegung der Clipbranchen 4, 6 bzw. die elastischen Aufweitung der Spiralfeder 14 stoppt. Ein Überspannen der Spiralfeder 14 wird somit verhindert.

Gemäß der vorstehenden Beschreibung lassen sich die besonderen Merkmale des erfindungsgemäßen chirurgischen Clip wie Folgt zusammenfassen:
Er besteht aus mindestens zwei, vorzugsweise drei Einzelbauteilen, nämlich den beiden Clipbranchen 1, 2 mit daran ausgebildeter/fixierter Spiralfeder 14 bzw. Lagerachse 8 sowie dem drehgesicherten Bolzen 12 zur Drehmomentübertragung zwischen den Branchen 1, 2, der zum Zusammenfügen der beiden Clipbranchen 1, 2 in die vorstehend beschriebenen Durchgangsöffnungen in der Spiralfeder 14 und der Lagerachse 8 eingesetzt und vorzugsweise reibschlüssig und/oder vorzugsweise durch Nieten, Kleben oder Schweißen fixiert wird. Bein Zusammenfügen dieser Bauteile kann dabei der Clip bzw. die beiden Clipbranchen 1, 2 mittels des Bolzens 14 in vorbestimmter Weise sowie auf einen bestimmten Wert vorgespannt werden.

Die verwendete Spiralfeder 14 erlaubt eine Optimierung der Federkraft und auch der Federgröße. Dabei ist es möglich, eine im Wesentlichen durchgehend konstante Biegespannung in der Spiralfeder 14 über deren vordefinierten Federweg (gemäß Platzierung des radialen Vorsprungs) zu realisieren und somit auch höhere und gleichmäßigere Schlusskräfte zu realisieren. Die maximale Öffnungsweite wird wie beschrieben, durch den Vorsprung oder Rücksprung 16 sicher begrenzt.

Die Lagerachse 8 und ggf. die sich daran anschließende Klemmschiene 4 kann durch MIM, CIM oder Schleuderguss hergestellt werden. Durch die Verwendung von keramischen Werkstoffen können Artefakte bei einer CT oder MRT vermieden werden. Auch können die bisher bekannten Maulteilgeometrien unterschiedlich zu den dargestellten Klemmschienen 4, 6 problemlos umgesetzt werden wie etwa Bajonett- oder Fenster-Geometrie.

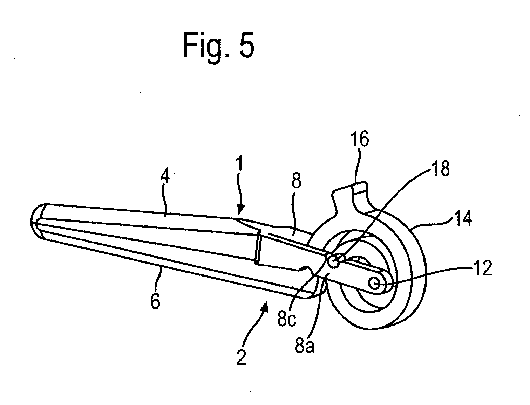

In der Fig. 5 ist ein chirurgischer Clip gemäß einem zweiten bevorzugten Ausführungsbeispiel der Erfindung gezeigt, der hinsichtlich des Aufbaus seiner Clipbranchen einschließlich der daran aus-/angeformten Spiralfeder bzw. Lagerachse dem chirurgischen Clip des ersten Ausführungsbeispiels im Wesentlichen entspricht, sich jedoch in der Bolzenform sowie in der Art der Drehmomentübertragung zwischen den beiden Clipbranchen vom ersten Ausführungsbeispiel unterscheidet. Aus diesem Grund werden nachfolgen im Wesentlichen nur die technischen/konstruktiven Unterschiede des Clips gemäß zweiten Ausführungsbeispiel näher beschrieben, wobei bezüglich der weiteren, im Wesentlichen übereinstimmenden Merkmale auf die vorstehende Beschreibung des Clip gemäß erstem Ausführungsbeispiel verwiesen wird. Im übrigen werden nachfolgend die gleichen Bezugszeichen für gleiche Bauteile verwendet.

Gemäß der Fig. 5 ist der Bolzen 12 zur Kopplung der Spiralfeder 14 (in deren Zentrum/Auge) mit der Lagerachse als runder Stift ausgebildet und daher per se nicht dafür vorgesehen ein Drehmoment zwischen den beiden Clipbranchen 1, 2 zu übertragen. D.h. in diesem Fall würde ein Aufweiten der Spiralfeder 14 durch Auseinanderziehen der beiden Klemmschienen 4, 6 unterbleiben, da der stiftförmige runde Bolzen 12 innerhalb der dementsprechend ebenfalls runden Durchgangsöffnungen in der Spiralfeder 14 und der Lagerachse 8 durchrutschen würde. Zwar wäre es denkbar, den Bolzen 12 derart reibschlüssig in die Durchgangsbohren zu pressen, dass die Drehmomentübertragung gewährleistet ist. Dies ist jedoch bei den geringen Abmessungen des Clip und damit des Bolzens 12 problematisch dahingehend, dass dieser beim Einpressvorgang zerbrechen oder verbiegen könnte.

Daher ist bei dem chirurgischen Clip gemäß der Fig. 5 ein vom Bolzen 12 radial (mit Bezug auf die Spiralfeder 14) beabstandeter Anschlagsstift 18 vorgesehen, der unter Ausbildung eines Art Hebelmechanismus an einer bestimmten Position an der Spiralfeder 14 fixiert / ausgebildet ist. Dieser Anschlagstift 18 kommt dabei zumindest bei einem Aufschwenken/Aufklappen der beiden Klemmschienen 4, 6 mit der Lagerachse 8 in Anlage, wodurch in Zusammenwirken der beiden Anlenkpunkte bestehend aus Bolzen 12 und Anschlagstift 18 ein Drehmoment zwischen den beiden Clipbranchen 1, 2 unter Aufweiten der Spiralfeder 14 übertragbar ist. Ferner hat die Lagerachse 8 im Anschlagsbereich mit dem Anschlagsstift 18 eine Auskerbung 8c, in die der Anschlagsstift 18 eingreift. Diese verhindert ein Abgleiten des Anschlagsstift 18 längs der Lagerachse 8, sodass die elastische Aufweitung der Spiralfeder 14 in einer vorbestimmten sowie kontrollierten Weise erfolgt.

Abschließend sei darauf hingewiesen, dass die in Konstruktionslage gemäß der Fig. 5 erreichte Vorspannung der Spiralfeder 14 von der Position des Anschlagstifts 18 längs der Spiralfeder abhängig ist. Es besteht daher die Möglichkeit, in der Spiralfeder 14 eine Anzahl von Sacklöchern längs der Federwindungen vorzusehen, in die der Anschlagstift 18 in ausgewählter Weise einsetzbar ist, um so unterschiedlich Vorspannungen einzustellen.

Offenbart wird ein chirurgischer Clip aus mindestens zwei Einzelbauteilen, umfassend eine erste, vorzugsweise eine Klemmschiene aufweisende Branche, an deren einem proximalen Endbereich ein Gabel- oder Ösenabschnitt oder ein Anlenkarm an-/ausgeformt oder anschließbar ist und eine hierzu separate zweite, vorzugsweise eine Klemmschiene aufweisende Branche, an deren einem proximalen Endbereich eine Spiralfeder an-/ausgeformt oder anschließbar ist, die in den Gabel-/Ösenabschnitt eingesetzt oder längs des Anlenkarms geführt ist und die mit dem Gabel-/Ösenabschnitt oder dem Anlenkarm vorzugsweise im Wesentlichen in deren Zentrum/Auge drehfest gekoppelt oder koppelbar ist.

## Patentansprüche

1. Chirurgischer Clip mit mindestens zwei Einzelbauteilen, umfassend eine erste, vorzugsweise eine Klemmschiene (4) aufweisende Branche (1) und eine hierzu separate zweite, vorzugsweise eine Klemmschiene (6) aufweisende Branche (2), wobei die erste Branche (1) einen Gabel- oder Ösenabschnitt oder einen Anlenkarm (8) aufweist, der an der ersten Branche (1) vorzugsweise an deren einem proximalen Endbereich, an-/ausgeformt oder anschließbar ist, **dadurch gekennzeichnet, dass** die zweite Branche (2) eine Spiralfeder (14) aufweist, die an ihrem radial äußerem Ende an der zweiten Branche (2) vorzugsweise an deren einem proximalen Endbereich an-/ausgeformt oder anschließbar ist, wobei die Spiralfeder (14) in den Gabel-/Ösenabschnitt (8) seitwärts eingesetzt oder längs des Anlenkarms seitlich geführt ist und mit dem Gabel-/Ösenabschnitt oder dem Anlenkarm (8) in deren Zentrum/Auge gekoppelt oder koppelbar ist.

2. Chirurgischer Clip nach Anspruch 1, **dadurch gekennzeichnet, dass** die Spiralfeder (14) zumindest in deren Aufweitrichtung drehfest für eine Drehmomentübertragung mit dem Gabel-/Ösenabschnitt oder Anlenkarm (8) gekoppelt ist.

3. Chirurgischer Clip nach Anspruch 2, **dadurch gekennzeichnet, dass** an der Spiralfeder (14) ein Rastvor-/-rücksprung (16) ausgeformt oder angeordnet ist, der zur Definition einer maximal zulässigen Deformation oder Aufweitung mit einem Anschlag an der ersten Branche (1) in Anlage bringbar ist, die am radial inneren Ende/Zentrum der Spiralfeder (14) angekoppelt ist.

4. Chirurgischer Clip nach Anspruch 3 **dadurch gekennzeichnet, dass** die Verbindung zwischen der zweiten Branche (2) und der Spiralfeder (14) in deren innerem Ende/Zentrum mittels zumindest eines Bolzens (12) erfolgt, der in übereinander sich anordnender Durchgangsöffnungen (10) im Zentrum der Spiralfeder (14) sowie in der zweiten Branche (2) eingesteckt oder einsteckbar ist und der einen in zumindest eine Drehrichtung wirkenden Formschluss mit der Spiralfeder (14) und der zweiten Branche (2) eingeht.

5. Chirurgischer Clip nach einem der Ansprüche 3 bis 4, **dadurch gekennzeichnet, dass** die Kopplung zwischen der zweiten Branche (2) und der Spiralfeder (14) in deren innerem Ende/Zentrum mittels zumindest eines Bolzens (12) erfolgt, der in übereinander sich anordnender Durchgangsöffnungen (10) im Zentrum der Spiralfeder (14) sowie in der zweiten Branche (2) eingesteckt oder einsteckbar ist, wobei ein radial vom Bolzen (12) beabstandeter Anschlagsvorsprung (18) an der Spiralfeder (14) vorgesehen ist, der einen zumindest in Aufweitrichtung der Spiralfeder (14) wirkenden Formschluss mit der zweiten Branche (2) eingeht.

6. Chirurgischer Clip nach einem der vorstehenden Ansprüche, **dadurch gekennzeichnet, dass** der Gabel- oder Ösenabschnitt (8) einen Aufnahmespalt für die Spiralfeder (14) ausbildet, dessen Spaltweite so dimensioniert ist, dass die Spiralfeder (14) seitlich in Gleitkontakt mit den Gabel- oder Ösenabschnitt (8) kommt oder ein geringes Spaltmaß ausbildet, derart, dass der Gabel- oder Ösenabschnitt (8) eine Führungs- und/oder Abstützfunktion für die Spiralfeder (14) ausübt.

7. Chirurgischer Clip nach Anspruch 5, **dadurch gekennzeichnet, dass** die quer verlaufenden Durchgangsöffnungen (10) in der Spiralfeder (14) und dem Gabel-/Ösenabschnitt oder dem Anlenkarm (8) ein unrundes Querschnittsprofil für eine Formschlussverbindung in Aufweitrichtung der Feder aufweisen.

8. Chirurgischer Clip nach Anspruch 7, **dadurch gekennzeichnet, dass** in unvorgespannter Konstruktionslage der ineinander gesteckten Branchen (1, 2) die unrunde Durchgangsöffnung in der Spiralfeder (14) um ein vorbestimmtes Winkelmaß verdreht zu den unrunden Durchgangsöffnungen im Gabel-/Ösenabschnitt oder Anlenkarm (8) ist, derart, dass ein gemeinsames Durchdringen aller Durchgangsöffnungen mittels eines entsprechend unrunden Bolzens (12) nur durch vorspannendes Verdrehen der Spiralfeder (14) zur Erreichung einer Deckungslage ermöglicht ist.

9. Chirurgischer Clip nach Anspruch 7 oder 8, **dadurch gekennzeichnet, dass** die unrunden Durchgangsöffnungen ein solches Profil vorzugsweise ein Vielnutprofil aufweisen, das unterschiedliche Drehpositionen insbesondere der unrunden Durchgangsöffnung der Spiralfeder (14) bezüglich der unrunden Durchgangsöffnungen im Gabel-/Ösenabschnitt oder Anlenkarm (8) für ein gemeinsames Durchdringen aller Durchgangsöffnungen mittels eines Bolzens (12) mit Vielnutprofil erlaubt, um so variabel einstellbare Vorspannungen zu ermöglichen.

10. Chirurgischer Clip nach einem der vorstehenden Ansprüche, **dadurch gekennzeichnet, dass** die Spiralfeder (14) so dimensioniert ist, dass eine im Wesentlichen durchgehend konstante Biegespannung innerhalb deren vorgesehenen Deformationswegs realisiert wird.

11. Chirurgischer Clip nach einem der vorstehenden Ansprüche, **dadurch gekennzeichnet, dass** die Spiralfeder (14) einstückig mit der zweiten Clipbranche (2) vorzugsweise durch ein Laser-Schneidverfahren oder ein Stanzverfahren ausgebildet ist.

12. Chirurgischer Clip nach einem der vorstehenden Ansprüche, **dadurch gekennzeichnet, dass** der Federdrahtquerschnitt der Spiralfeder (14) eine Rechtecksform hat.

## Claims

1. A surgical clip having at least two individual components, comprising a first branch (1) preferably having a clamping rail (4), and separate therefrom a second branch (2) preferably having a clamping rail (6), the first branch (1) having a fork or eyelet portion or a link arm (8) which is integrally formed / shaped on or connectable to the first branch (1) preferably at the one proximal end region thereof, **characterised in that** the second branch (2) has a spiral spring (14) which at its radially outer end is integrally formed / shaped on or connectable to the second branch (2) preferably at the one proximal end region thereof, the spiral spring (14) being inserted sideways into the fork/eyelet portion (8) or being laterally guided along the link arm and being coupled or capable of being coupled to the fork/eyelet portion or the link arm (8) in the centre/eye thereof.

2. A surgical clip according to claim 1, **characterised in that** the spiral spring (14) is coupled to the fork/eyelet portion or link arm (8) in a manner securing it against relative rotation, at least in its expanding direction, for transmission of torque.

3. A surgical clip according to claim 2, **characterised in that** there is shaped on or disposed on the spiral spring (14) a locking projection/recess (16) which for definition of a maximum permissible deformation or expansion may be brought into contact with a stop on the first branch (1) which is coupled to the radially inner end/centre of the spiral spring (14).

4. A surgical clip according to claim 3, **characterised in that** the connection between the second branch (2) and the spiral spring (14) is made in the inner end/centre thereof by means of at least one pin (12) which is inserted or insertable into passage openings (10) coming to lie over one another in the centre of the spiral spring (14) and in the second branch (2) and which enters into a form-fit with the spiral spring (14) and the second branch (2), which form-fit is effective at least in one direction of rotation.

5. A surgical clip according to one of claims 3 and 4, **characterised in that** the coupling between the second branch (2) and the spiral spring (14) is made in the inner end/centre thereof by means of at least one pin (12) which is inserted or insertable into passage openings (10) coming to lie over one another in the centre of the spiral spring (14) and in the second branch (2), there being provided on the spiral spring (14) a stop projection (18) which is radially spaced from the pin (12) and which enters into a form-fit with the second branch (2), which form-fit is effective at least in the expanding direction of the spiral spring (14).

6. A surgical clip according to any one of the preceding claims, **characterised in that** the fork or eyelet portion (8) forms a receiving gap for the spiral spring (14), the gap width of which is so dimensioned that the spiral spring (14) comes laterally into sliding contact with the fork or eyelet portion (8) or provides a slight clearance in such a manner that the fork or eyelet portion (8) performs a guiding and/or supporting function for the spiral spring (14).

7. A surgical clip according to claim 5, **characterised in that** the transversely extending passage openings (10) in the spiral spring (14) and the fork/eyelet portion or the link arm (8) have an out-of-round cross-sectional profile for a form-fit connection in the expanding direction of the spring.

8. A surgical clip according to claim 7, **characterised in that**, in the non-pretensioned construction position of the branches (1, 2) inserted one into the other, the out-of-round passage opening in the spiral spring (14) is rotated relative to the out-of-round passage openings in the fork/eyelet portion or link arm (8) by a predetermined angular dimension in such a manner that penetration of all the passage openings together by means of a correspondingly out-of-round pin (12) is made possible only by pretensioning rotation of the spiral spring (14) to reach a registration position.

9. A surgical clip according to claim 7 or 8, **characterised in that** the out-of-round passage openings have such a profile, preferably a multiple-groove profile, that different rotational positions especially of the out-of-round passage opening of the spiral spring (14) relative to the out-of-round passage openings in the fork/eyelet portion or link arm (8) allow penetration of all the passage openings together by means of a pin (12) of multiple-groove profile in order to make variably adjustable pretensions possible **in that** manner.

10. A surgical clip according to any one of the preceding claims, **characterised in that** the spiral spring (14) is so dimensioned that a bending stress that is substantially constant throughout is obtained within its intended deformation travel.

11. A surgical clip according to any one of the preceding claims, **characterised in that** the spiral spring (14) is formed in one piece with the second clip branch (2) preferably by a laser cutting process or a punching process.

12. A surgical clip according to any one of the preceding claims, **characterised in that** the spring wire cross-section of the spiral spring (14) is of a rectangular shape.

## Revendications

1. Clip chirurgical comportant au moins deux éléments individuels, comprenant une première branche (1) présentant de préférence une barre de serrage (4) et une deuxième branche (2) séparée de celle-ci, présentant de préférence une barre de serrage (6), dans lequel la première branche (1) présente un segment en fourche ou en oeillet ou un bras d'articulation (8) qui est monté/formé ou qui peut être relié à la première branche (1), de préférence à une extrémité proximale de celle-ci, **caractérisé en ce que** la deuxième branche (2) présente un ressort hélicoïdal (14) qui est monté/formé ou qui peut être relié au niveau de son extrémité externe radiale, à la deuxième branche (2) de préférence à une extrémité proximale de celle-ci, dans lequel le ressort hélicoïdal (14) est inséré latéralement dans le segment en fourche/ en oeillet (8) ou est conduit le long du bras d'articulation et est couplé ou peut être couplé au segment en fourche/ en oeillet ou au bras d'articulation (8) dans leur centre/oeil.

2. Clip chirurgical selon la revendication 1, **caractérisé en ce que** le ressort hélicoïdal (14) est couplé sans pouvoir tourner, au moins dans sa direction d'élargissement, pour une transmission du moment de rotation, au segment en fourche/ en oeillet ou au bras d'articulation (8).

3. Clip chirurgical selon la revendication 2, **caractérisé en ce que** sur le ressort hélicoïdal (14) est formé(e) ou est disposé(e) une saillie/un renfoncement d'encliquetage (16) qui peut être amené(e) en contact avec une butée sur la première branche (1) qui est couplée à l'extrémité interne radiale/au centre du ressort hélicoïdal (14) pour la définition d'une déformation ou d'une ouverture maximale autorisée.

4. Clip chirurgical selon la revendication 3, **caractérisé en ce que** la liaison entre la deuxième branche (2) et le ressort hélicoïdal (14) s'effectue dans leur extrémité interne/centre au moyen d'au moins un boulon (12) qui est fiché ou peut être fiché dans des ouvertures de passage disposées les unes sur les autres (10) au centre du ressort hélicoïdal (14) et dans la deuxième branche (2) et qui établit une liaison agissant dans au moins un sens de rotation avec le ressort hélicoïdal (14) et la deuxième branche (2).

5. Clip chirurgical selon l'une des revendications 3 à 4, **caractérisé en ce que** le couplage s'effectue entre la deuxième branche (2) et le ressort hélicoïdal (14) dans leur extrémité interne/centre au moyen d'au moins un boulon (12) qui est fiché ou peut être fiché dans des ouvertures de passage disposées les unes sur les autres (10) au centre du ressort hélicoïdal (14) et dans la deuxième branche (2), dans lequel une saillie de butée (18) distante radialement du boulon (12) est prévue sur le ressort hélicoïdal (14) qui établit une liaison agissant au moins dans la direction d'élargissement du ressort hélicoïdal (14) avec la deuxième branche (2).

6. Clip chirurgical selon l'une des revendications précédentes, **caractérisé en ce que** le segment en fourche ou en oeillet (8) forme une fente de réception pour le ressort hélicoïdal (14) dont la largeur est dimensionnée de telle sorte que le ressort hélicoïdal (14) vient latéralement en contact coulissant avec le segment en fourche ou en oeillet (8) ou forme une dimension de fente réduite de telle sorte que le segment en fourche et/ou en oeillet (8) exerce une fonction de guidage et/ou d'appui pour le ressort hélicoïdal (14).

7. Clip chirurgical selon la revendication 5, **caractérisé en ce que** les ouvertures de passage (10) évoluant perpendiculairement dans le ressort hélicoïdal (14) et le segment en fourche/ en oeillet ou le bras d'articulation (8) présentent un profil de section non arrondi pour une liaison solidaire dans la direction d'élargissement du ressort.

8. Clip chirurgical selon la revendication 7, **caractérisé en ce que**, à l'état de construction non précontraint des branches emboîtées l'une dans l'autre (1, 2), l'ouverture de passage non arrondie dans le ressort hélicoïdal (14) tourne d'un angle prédéterminé vers les ouvertures de passage non arrondies dans le segment en fourche/ en oeillet ou le bras d'articulation (8) de telle sorte qu'une pénétration commune de toutes les ouvertures de passage peut être réalisée au moyen d'un boulon non arrondi correspondant (12) uniquement par la rotation précontrainte du ressort hélicoïdal (14) pour atteindre un recouvrement.

9. Clip chirurgical selon la revendication 7 ou 8, **caractérisé en ce que** les ouvertures de passage non arrondies présentent ce profil, de préférence, un profil cannelé qui permet des positions de rotation différentes en particulier de l'ouverture de passage non arrondie du ressort hélicoïdal (14) par rapport aux ouvertures de passage non arrondies dans le segment en fourche/ en oeillet ou le bras d'articulation (8) pour une pénétration commune de toutes les ouvertures de passage au moyen d'un boulon (12) avec un profil cannelé pour permettre ainsi des précontraintes ajustables de façon variable.

10. Clip chirurgical selon l'une des revendications précédentes, **caractérisé en ce que** le ressort hélicoïdal (14) est dimensionné de telle sorte qu'une contrainte de flexion essentiellement continuellement constante est réalisée dans sa voie de déformation prévue.

11. Clip chirurgical selon l'une des revendications précédentes, **caractérisé en ce que** le ressort hélicoïdal (14) est formé d'un seul tenant avec la deuxième branche du clip (2) de préférence par un procédé de découpe laser ou un procédé d'estampage.

12. Clip chirurgical selon l'une des revendications précédentes, **caractérisé en ce que** la section du fil de ressort du ressort hélicoïdal (14) a une forme rectangulaire.
